# EUROPEAN PATENT APPLICATION

(11) **EP 2 645 105 A1**
(43) Date of publication of application: **02.10.2013**
(21) Application number: 12161217.0
(22) Date of filing: 26.03.2012
(51) Int. Cl.: G01N 33/68

(54) **Early biomarkers of age-related low-grade inflammation**

(71) Applicant: Nestec S.A., 1800 Vevey (CH); INRA, 75338 Paris (FR)
(72) Inventor: Brahmbhatt, Viral, CH-1066 Epalinges (CH); Breuille, Denis, CH-1010 Lausanne (CH); Guy, Philippe Alexandre, CH-1522 Lucens (CH); Montoliu Roura, Ivan, CH-1000 Lausanne 26 (CH); Papet, Isabelle, 63100 Clermont-Ferrand (FR); Vidal, Karine, CH-1010 Lausanne (CH)
(74) Representative: Chautard, Cécile

(57) **Abstract**

The present invention relates to a method for predicting the risk of acquiring an age-related low-grade inflammation for a subject, said method comprising a) providing a biological sample from a subject, b) determining in said sample the level of at least one biomarker selected from the group consisting of a compound of molecular weight between 859-863 g/mol and which is an alkylacylphosphatidylcholine, a compound of molecular weight between 861-865 g/mol and which is a diacylphosphatidylcholine, a compound of molecular weight between 791-794 g/mol and which is an alkylacylphosphatidylcholine, a compound of molecular weight between 522-525 g/mol and which is a monoacylphosphatidylcholine, octadecanoylcarnitine (C18), and tryptophan, c) comparing the level of the at least one biomarker to a reference level, and d) determining whether said subject is likely to be at risk of acquiring an age-related low-grade inflammation, when the level of biomarker(s) deviate significantly from the respective reference level.

## Description

### Technical field of the invention

The present invention relates to biomarkers for predicting the risk of a subject to acquire age-related low-grade inflammation.

### Background of the invention

The aging of population in developed countries generates a socioeconomic problem related to the growing number of frail and dependent persons. Aging has been associated with increased levels of (chronic) low-grade inflammation. Persistent inflammatory conditions are considered as a risk factor for age-associated conditions such as type 2 diabetes, hypertension, ischemic heart disease, atherosclerosis, Irritable Bowel Syndrome, Inflammatory Bowel Disease, psoriasis, cystic fibrosis, osteoporosis, osteoarthritis rheumatoid arthritis, sarcopenia, steatohepatitis, non alcoholic fatty liver disease, Alzheimer's disease, and Parkinson's disease. Consistent with this, inflammatory mediators are associated with frailty syndrome and related morbidity in elderly subjects.

Management of inflammation has been associated with "successful" aging. To assess inflammatory status, several indicators such as blood levels of inflammatory markers (e.g. α-2-macroglobulin, fibrinogen and albumin) are typically monitored.

For example, increases in C-reactive protein (CRP), which is a widely used marker of inflammation, can predict cardiovascular risk and risk for impaired cognition. However, a drawback of CRP and other markers is however that they increase only after the onset of inflammation. Hence, it is likely that irreversible damage has already occurred at this point and the chances of success for preventive remedies are low beyond this stage. Beside these markers, metabolic approaches would be highly valuable in providing characteristic metabolic signature linked to the progression of inflammation. Metabolite profiling of body fluids may be a minor invasive method to monitor physiological or pathophysiological changes in a given subject as a result of disease progression, pharmacological or nutritional intervention. Metabolites are small molecules that reflect whole body metabolic processes and have been positioned as the most representative measures of a given phenotype.

Hence, an improved method for assessing the risk for acquiring (chronic) low-grade inflammation would be advantageous.

### Summary of the invention

The present inventors have identified a specific set of biomarkers which may be used for predicting the risk of acquiring an age-related low-grade inflammation for a subject. This may be particularly useful for identifying subjects at an early stage before the onset of a potential chronic inflammation. As mentioned above other inflammation biomarkers are present after the onset of the low-grade inflammation, or at least only present when the inflammation may also be determined by other clinical means.

Thus, an object of the present invention relates to the provision of early biomarkers for age-related low-grade inflammation.

In particular, it is an object of the present invention to provide a set of biomarkers that solve the above mentioned problems of the prior art with detection after onset of inflammation.

Thus, one aspect of the invention relates to a method for predicting the risk of acquiring an age-related low-grade inflammation for a subject, said method comprising
a) providing a biological sample from a subject,
b) determining in said sample the level of at least one biomarker selected from the group consisting of
   - a compound of molecular weight between 859-863 g/mol and which is an alkylacylphosphatidylcholine,
   - a compound of molecular weight between 861-865 g/mol and which is a diacylphosphatidylcholine,
   - a compound of molecular weight between 791-794 g/mol and which is an alkylacylphosphatidylcholine,
   - a compound of molecular weight between 522-525 g/mol and which is a monoacylphosphatidylcholine,
   - octadecanoylcarnitine (C18), and
   - tryptophan
c) comparing the level of the at least one biomarker to a reference level, and
d) determining whether said subject is likely to be at risk of acquiring an age-related low-grade inflammation, when the level of biomarker(s) deviate significantly from the respective reference level.

Another aspect of the present invention relates to a method for determining the effect of a treatment for lowering the risk of acquiring an age-related low-grade inflammation for a subject, said method comprising
- providing a first biological sample from the subject, obtained before a treatment, and determining a first risk of acquiring an age-related low-grade inflammation according to the present invention,
- providing a second biological sample from the subject, obtained subsequent to the treatment or during the treatment, and determining a second risk of acquiring an age-related low-grade inflammation according to any the present invention, and
- comparing the first risk to the second risk, thereby providing an estimate of the effect of the treatment.

### Brief description of the figures

Figures 1-3 show early indicators of the onset of inflammation. Selected metabolites from NItoI animals (animal evolved from non-inflamed to inflamed during the experiment) under alanine diet. Plotted concentration Median values (solid line) and their bootstrapped confidence intervals (dotted lines). Concentration units (y axes) were expressed in µM.
Figures 4-6 show boxplots at time points 1 and 6 (-13-14 weeks) on the six metabolites, between permanently non-inflamed (n=12), non-inflamed to inflamed (evolving) and inflamed (n=7) animals. Concentration unit was expressed in µM.

The present invention will now be described in more detail in the following.

### Detailed description of the invention

In a first aspect the invention relates to a method for predicting the risk of acquiring an age-related low-grade inflammation for a subject, said method comprising
a) providing a biological sample from a subject,
b) determining in said sample the level of at least one biomarker selected from the group consisting of
   - a compound of molecular weight between 859-863 g/mol and which is an alkylacylphosphatidylcholine,
   - a compound of molecular weight between 861-865 g/mol and which is a diacylphosphatidylcholine,
   - a compound of molecular weight between 791-794 g/mol and which is an alkylacylphosphatidylcholine,
   - a compound of molecular weight between 522-525 g/mol and which is a monoacylphosphatidylcholine,
   - octadecanoylcarnitine (C18), and
   - tryptophan
c) comparing the level of the at least one biomarker to a reference level,
d) determining whether said subject is likely to be at risk of acquiring an age-related low-grade inflammation, when the level of biomarker(s) deviate significantly from the respective reference level.

In the present context the term "low grade inflammation" is characterized by increased levels of one or more of acute phase response biomarkers (e.g. C-reactive protein, serum amyloid A (SAA), orosomucoid, fibrinogen); and/or elevated levels of one or more of a pro-inflammatory cytokine (e.g., IL-6, TNF alpha, IFN gamma); and/or increased plasma viscosity, erythrocyte sedimentation rate (ESR) and/or leukocyte count; and/or decreased levels of serum albumin, as compared to a reference value and in the absence of observable inflammation (e.g. redness, swelling, pain).

In a particular embodiment, low grade inflammation is characterized by elevation of one or more of the mentioned markers. In yet a particular embodiment, low-grade inflammation may be characterised as an hs-CRP level of higher than 1 mg/l (particularly more than 3 mg/l), for example as measured using the particle enhanced immunoturbidimetry assay (Roche Diagnostics). These particular embodiments may be more relevant when the subject is a human.

In an embodiment the compound of molecular weight between 859-863 g/mol and which is an alkylacylphosphatidylcholine is phosphatidylcholine (PC(O-42:0)).

In another embodiment the compound of molecular weight between 861-865 g/mol and which is a diacylphosphatidylcholine is phosphatidylcholine (PC(42:6)).

In yet an embodiment the compound of molecular weight between 791-794 g/mol and which is an alkylacylphosphatidylcholine is phosphatidylcholine (PC(O-38:6)).

In a further embodiment the compound of molecular weight between 522-525 g/mol and which is a monoacylphosphatidylcholine is lysophosphatidylcholine (LPC(18:0)).

In general, it is to be understood that the above list of compounds may also include salts or adducts of the compounds.

The change in the levels of the biomarkers according to the invention (compared to the reference level) may be different between different subgroups of the listed biomarkers when the risk is evaluated. Thus, in an embodiment the one or more biomarkers are selected from the group consisting of a compound of molecular weight between 859-863 g/mol and which is an alkylacylphosphatidylcholine, a compound of molecular weight between 861-865 g/mol and which is a diacylphosphatidylcholine, a compound of molecular weight between 791-794 g/mol and which is an alkylacylphosphatidylcholine, a compound of molecular weight between 522-525 g/mol and which is a monoacylphosphatidylcholine, and octadecanoylcarnitine (C18),
whereby said subject is likely to be at risk of acquiring an age-related low-grade inflammation, if said determined one or more levels are significantly higher than the reference level and/or said subject is unlikely to be at risk of acquiring an age-related low-grade inflammation, if said determined one or more levels are equal to or lower than the reference level.

In another embodiment the biomarker is phosphatidylcholine (PC(O-42:0)), phosphatidylcholine (PC(42:6)), lysophosphatidylcholine (LPC(18:0)), phosphatidylcholine (PC(O-38:6)), or octadecanoylcarnitine (C18).

In another embodiment the biomarker is tryptophan,
whereby said subject is likely to be at risk of acquiring an age-related low-grade inflammation, if said determined the level is significantly lower than the reference level and/or said subject is unlikely to be at risk of acquiring an age-related low-grade inflammation, if said determined the level is equal to or higher than the reference level. In a further embodiment the biomarker is tryptophan.

To improve the strength of the analysis it may be advantageous to determine the level of more than one biomarker. Thus, in an embodiment the level of at least two biomarkers are determined, such as level of at least three, such as level of at least four, such as level of at least five, or such as the level of all six biomarkers are determined.

In yet an embodiment at least the level of one of a compound of molecular weight between 859-863 g/mol and which is an alkylacylphosphatidylcholine, a compound of molecular weight between 861-865 g/mol and which is a diacylphosphatidylcholine, a compound of molecular weight between 791-794 g/mol and which is an alkylacylphosphatidylcholine, is determined. In yet another embodiment the levels of at least two biomarkers selected from the group consisting of a compound of molecular weight between 859-863 g/mol and which is an alkylacylphosphatidylcholine, a compound of molecular weight between 861-865 g/mol and which is a diacylphosphatidylcholine, a compound of molecular weight between 791-794 g/mol and which is an alkylacylphosphatidylcholine, are determined, such as all three biomarkers.

To determine whether the subject has a risk above normal, a cut-off must be established (reference level). This cut-off may be established by the laboratory, the physician or on a case by case basis by each subject.

The cut-off level could be established using a number of methods, including: percentiles, mean plus or minus standard deviation(s); multiples of median value; patient specific risk or other methods known to those who are skilled in the art.

The multivariate discriminant analysis and other risk assessments can be performed on the commercially available computer program statistical package Statistical Analysis system (manufactured and sold by SAS Institute Inc.) or by other methods of multivariate statistical analysis or other statistical software packages or screening software known to those skilled in the art.

As obvious to one skilled in the art, in any of the embodiments discussed above, changing the risk cut-off level of a positive, or using different a priori risks which may apply to different subgroups in the population, could change the results of the discriminant analysis for each subject.

When levels of specific biomarkers in a sample (such as a blood plasma sample) are compared to a reference level they can either be different (above or below the reference value) or equal. However, using today's detection techniques an exact definition of different or equal result can be difficult because of noise and variations in obtained expression levels from different samples. Hence, the usual method for evaluating whether two or more levels are different or equal involves statistics.

Statistics enables evaluation of significantly different expression levels and significantly equal expressions levels. Statistical methods involve applying a function/statistical algorithm to a set of data. Statistical theory defines a statistic as a function of a sample where the function itself is independent of the sample's distribution: the term is used both for the function and for the value of the function on a given sample. Commonly used statistical tests or methods applied to a data set include t-test, f-test or even more advanced test and methods of comparing data. Using such a test or methods enables a conclusion of whether two or more samples are significantly different or significantly equal.

The significance may be determined by the standard statistical methodology known by the person skilled in the art.

In an embodiment the reference level is an average value of a non-inflamed group of subjects. In another embodiment the reference level is the level determined for the same subject at one or more earlier points in time and wherein the subject was determined not to be inflamed.

The chosen reference level may be changed depending on the subject for which the test is applied. The chosen reference level may be changed if desiring a different specificity or sensitivity as known in the art.

Thus, in an embodiment said reference level is determined based on a desired sensitivity and specificity.

As used herein the sensitivity refers to the measures of the proportion of actual positives which are correctly identified as such - in analogy with a diagnostic test, i.e. the percentage of subjects being identified as at risk of being predisposed to aging related low-grade inflammation. Usually the sensitivity of a test can be described as the proportion of true positives of the total number with the target disorder. All subjects with the target disorder are the sum of (detected) true positives (TP) and (undetected) false negatives (FN).

As used herein the specificity refers to measures of the proportion of negatives which are correctly identified - i.e. the percentage of subjects being identified as having a normal risk of being predisposed to aging related low-grade inflammation. The ideal diagnostic test is a test that has 100 % specificity, i.e. only detects subjects with higher risk of being predisposed to aging related low-grade inflammation and therefore no false positive results, and 100 % sensitivity, i.e. detects all mammals being predisposed to aging related low-grade inflammation and therefore no false negative results.

For any test, there is usually a trade-off between each measure. For example in a manufacturing setting in which one is testing for faults, one may be willing to risk discarding functioning components (low specificity), in order to increase the chance of identifying nearly all faulty components (high sensitivity). This trade-off can be represented graphically using a ROC curve.

Selecting a sensitivity and specificity it is possible to obtain the optimal outcome in a detection method. In determining the discriminating value distinguishing mammals having a fertility potential below normal, the person skilled in the art has to predetermine the level of specificity. The ideal diagnostic test is a test that has 100% specificity, and therefore no false positive results, and 100% sensitivity, and therefore no false negative results. However, due to biological diversity no method can be expected to have 100% sensitive without including a substantial number of false negative results.

The chosen specificity determines the percentage of false positive cases that can be accepted in a given study/population and by a given institution. By decreasing specificity an increase in sensitivity is achieved. One example is a specificity of 95% which will result in a 5% rate of false positive cases.

As will be generally understood by those skilled in the art, methods for screening are processes of decision making and therefore the chosen specificity and sensitivity depends on what is considered to be the optimal outcome by a given institution/clinical personnel.

The (biological) sample of the invention refers to a sample obtained from a subject. The sample may be a biopsy or a body fluid sample, such as a blood sample. The sample may be processed before subjecting the sample to any of the methods of the invention. For example a sub-fraction of the sample may be isolated such as a blood plasma sample. Thus, in an embodiment said biological sample is a body fluid such as blood plasma, whole blood, blood serum, saliva and urine, or a tissue sample such as a tissue biopsy.

The level of the biomarkers may refer to different parameters such as concentration or activity. In an embodiment the level of biomarker is the concentration of the biomarker in the biological sample. The levels of the biomarkers in the biological sample may be assessed by different means. Thus, in an embodiment the levels of said biomarkers are determined by mass spectrometry, such as LC-ESI-MS/MS.

The risk of acquiring an age-related low-grade inflammation may be specified as the risk within a certain period from the time of sampling. Thus, in an embodiment the risk of acquiring an age-related low-grade inflammation is the risk within a period of 24 month from sampling, such as 12 months from sampling, such as within 8 months, such as within 6 months, such as within 4 months, such as within 2 months, such as within 1 month.

Low grade inflammations are present in subjects suffering from numerous conditions. Thus, in an embodiment the determined risk is also the risk of acquiring one or more conditions defined by a low-grade inflammation, such as age-related (chronic) low-grade inflammation. In a further embodiment the condition is selected from the group consisting of type 2 diabetes, hypertension, ischemic heart disease, atherosclerosis, Irritable Bowel Syndrome, Inflammatory Bowel Disease, psoriasis, cystic fibrosis, osteoporosis, osteoarthritis rheumatoid arthritis, sarcopenia, steatohepatitis, non alcoholic fatty liver disease, Alzheimer's disease, and Parkinson's disease. Thus, subjects tested to be at risk of acquiring an age-related low-grade inflammation, may also be considered at risk of acquiring one of the above listed conditions. Thus, subjects tested to be at risk of acquiring an age-related low-grade inflammation, may also be considered at risk of acquiring a chronic low-grade inflammation.

In the present context the term "subject" includes mammals such as humans or non-human species. The present invention has applicability, therefore, in human medicine as well as having livestock and veterinary and wild life applications. Thus, in a further embodiment the subject is a mammal such a human; a non-human species, including a primate; a livestock animal such as a sheep, a cows, a pig, a horse, a donkey, or a goat; a laboratory test animals such as mice, rats, rabbits, guinea pigs, or hamsters; or a companion animal such as a dog or a cat.

The term "age-related low-grade inflammation" refers to low grade inflammation in human subjects whose body function, for example in terms of metabolism and/or immunological status, has been affected as a result of advancing age. Thus, in an embodiment the subject is a human more than 20 years of age, such as more than 30 years of age, such as more than 40 years of, such as more than 50 years of age, such as more than 60 years of age, or such as more than 70 years of age. Generally such subjects will be more than 50 years of age. However, persons may suffer from diseases resulting in faster aging in terms of metabolism or immunological status.

Since treatments may be initiated to lower the risk of acquiring an age-related low-grade inflammation, it may be advantageous to be able to evaluate the effect of such treatment. Thus, in another aspect of the invention relates to a method for determining the effect of a treatment for lowering the risk of acquiring an age-related low-grade inflammation for a subject, said method comprising
- providing a first biological sample from the subject, obtained before a treatment, and determining a first risk of acquiring an age-related low-grade inflammation according to the present invention,
- providing a second biological sample from the subject, obtained subsequent to the treatment or during the treatment, and determining a second risk of acquiring an age-related low-grade inflammation according to the present invention, and
- comparing the first risk to the second risk, thereby providing an estimate of the effect of the treatment.

By evaluating changes in risk over time the effect of a treatment may be estimated. If required further biological samples may be provided which have been obtained at other time points. The determined changes in risk over time may dependent on the provided treatment and thus also assist in determining whether such treatment should be continued. It is to be understood that the treatment itself is not part of the present invention. Thus, in an embodiment
- a significantly increased risk in the second determined risk compared to the first determined risk is indicative of that the treatment is increasing the risk of acquiring an age-related low-grade inflammation, or
- an unchanged risk in the second determined risk compared to the first determined risk is indicative of that the treatment does not influence the risk of acquiring an age-related low-grade inflammation, or
- a significantly lower risk in the second determined risk compared to the first determined risk is indicative of that the treatment is lowering the risk of acquiring an age-related low-grade inflammation.

Different types of treatments by be evaluated using the method according to the present invention. In an embodiment the treatment is a dietary treatment or a pharmaceutical treatment. It is to be understood that the treatment itself does not form part of the present invention. The dietary treatment may be in the form of food products from animal and/or vegan sources, drinks, nutritional formula, compositions for clinical nutritions, nutritional powders to be reconstituted by addition of water, juice or milk, a nutritional, a food additive, a food component, a dairy product, or a gel, or a pet food product. Such products may be administrated orally, enterally or parenterally.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

### Example 1

Aging rats'model: Wistar rats were followed from the age of 18 to 24 months. Blood samples were collected at different time points for various analyses. At the age of 21 months, rats were divided in two groups, non-inflamed and inflamed as reflected by the measure of plasma α2-macroglobulin to evaluate low-grade inflammation.

Metabolomic analysis: Quantitative analysis of 163 metabolites, including acylcarnitines (n=41), amino acids (n=14), glycerophospholipids (n=92), sphingolipids (n=15), and hexose (n=1,C₆H₁₂O₆), have been realized in selected rat plasma samples using isotope dilution LC-MS/MS technique. Micromolar concentration levels of these metabolites were evaluated by multivariate regression against α2-macroglobulin (for each blood sampling) to evaluate potential correlation (and/or early predictive markers) of specific metabolites with well accepted clinical measurements. Statistical data treatment has been performed using non supervised and supervised approaches.

### Experimental study design

The animal study was conducted in accordance with the French National Research Council's Guidelines for the Care and Use of Laboratory Animals, complying with Nestlé animal welfare policy. Wistar male rats were bred in a conventional animal facility. When rats were 18 months of age, they were maintained in collective cages (3 to 4 per cage) under controlled conditions (temperature 21°C, relative humidity 55%, 12-h dark period starting at 20:00) with free access to water and standard diet. The standard diet in this period was composed of 16% protein, 3% fat, 60% carbohydrate, 12% water, fibers, vitamins, and minerals. Starting at the age of 21 months and for 14 weeks, rats were fed with alanine supplemented diet.

Blood samples were collected at specific time points for clinical measurements and metabonomics analysis. Rats exhibiting a blood concentration of α2-macroglobulin, higher than 82 mg/l were considered as low-grade inflamed, and those with α2-macroglobulin lower than 82 mg/l were considered as non-inflamed rats. To identify the early markers of age-related low-grade inflammation, rats initially non-inflamed (i.e. during the pre-experimental period) and rats becoming inflamed during the experimental period (i.e. from 21 months of age and the following 14 weeks) were studied. Overall, this corresponded to 16 animals. For validation purposes, metabolic data from animals permanently inflamed and permanently non-inflamed were also used.

### Metabolomics analysis

In total, 163 metabolites were quantified in rat plasma samples by LC-ESI-MS/MS using isotopically labeled internal standards (IS) (Biocrates Inc. Insbruck, Austria). Metabolite concentrations were calculated from the peak area ratio of analyte against known IS, generated via MetIQ software (Biocrates Inc.). As a standard pre-processing procedure of the quantitative data, a clean-up of the data was applied to remove the excess of unknowns for some variables. This step implied the replacement of the undetermined values (mainly associated with concentration below the limit of quantification (LOQ)) on each variable by the minimum of the variable and the complete removal of the variable if it initially presented more than a 15% of non determinations. Once this clean-up was completed, up to 6 metabolites were finally removed from the total list: five acylcarnitines (C3-OH, C3:1, C4:1, C5:1, C5:1-DC) and one lysophosphatidylcholine (lysoPC a C6:0). Prior to the multivariate analysis, all variables (metabolite concentrations), were centred and scaled to unit variance. To identify the pool of metabolites most likely to provide a metabolic signature associated to the onset of the inflammation, there were evaluated the outcomes of two multivariate regression models (Orthogonal Projections to Latent Structures, OPLS) between the plasma metabolite concentrations and the concentration of α2-macroglobulin (Log transformed). The models captured the metabolic changes in animals evolving from not inflamed to inflamed status, both in standard and alanine feeding conditions.

### Results

Relevant compounds with Variable Importance in Projection (VIP) above 0.9, common to both models, were considered as metabolic signature associated to the development of inflammation under the standard diet. This intersection provided up to 24 compounds. Furthermore, this set of metabolites was screened to determine if their concentrations were at a significantly different level between the beginning and the end of the experiment. With this aim, unpaired Mann-Whitney U test was applied to the selected metabolite data. Significance P values associated to each metabolite between the time points 1 (-13 weeks) and 6 (14 weeks) were calculated and later ranked according to their P value (table 1). As a threshold, it was chosen a P value below 0.20 to reduce the chance of false negatives (Type II error). This data is for the "evolving group" 13 animals.

**Table 1: Identified biomarkers having a P-value below 0.20.**

| Metabolite | P-value |
|---|---|
| Tryptophan | 0.000079 |
| PC(O-42:0) | 0.020770 |
| PC(42:6) | 0.036249 |
| LPC (18:0) | 0.091637 |
| Acylcarnitine C18 | 0.149156 |
| PC(O-38:6) | 0.165437 |

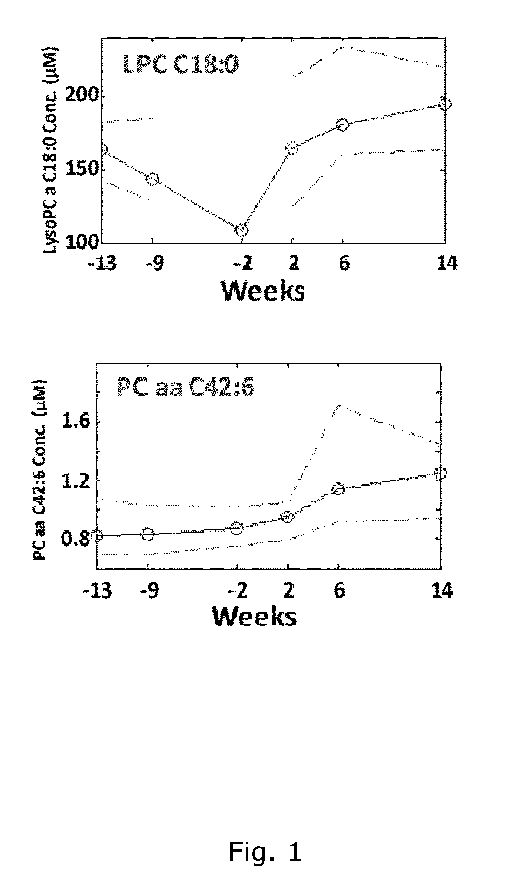

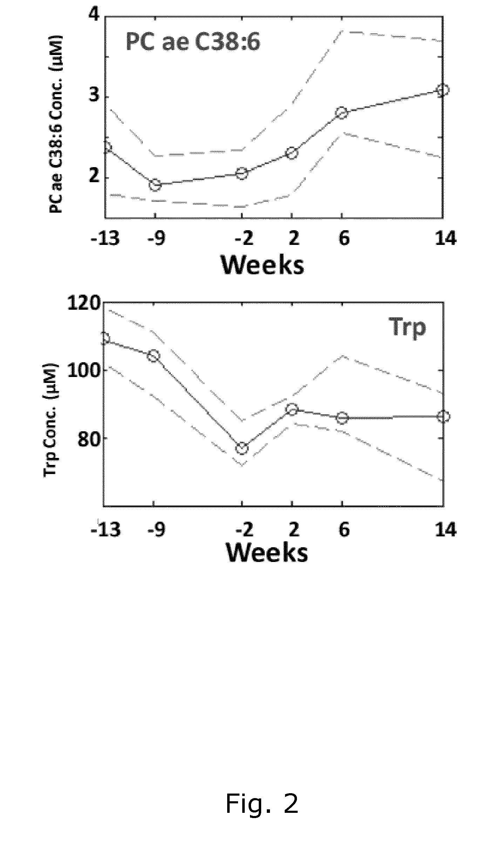

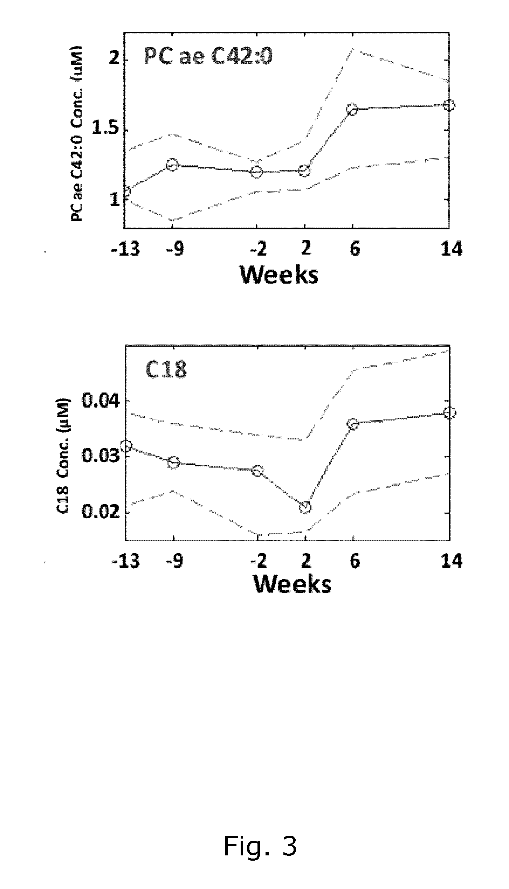

This selection reduced the list to 6 metabolites, including one amino acids (Trp), one acylcarnitines (C18), and four phospholipids (PC(O-42:0), PC(C42:6), LPC(18:0), and PC(O-38:6)). Their kinetic profiles were evaluated individually. All these compounds showed changes that could be qualitatively considered relevant along the experimental time (figures 1-3).

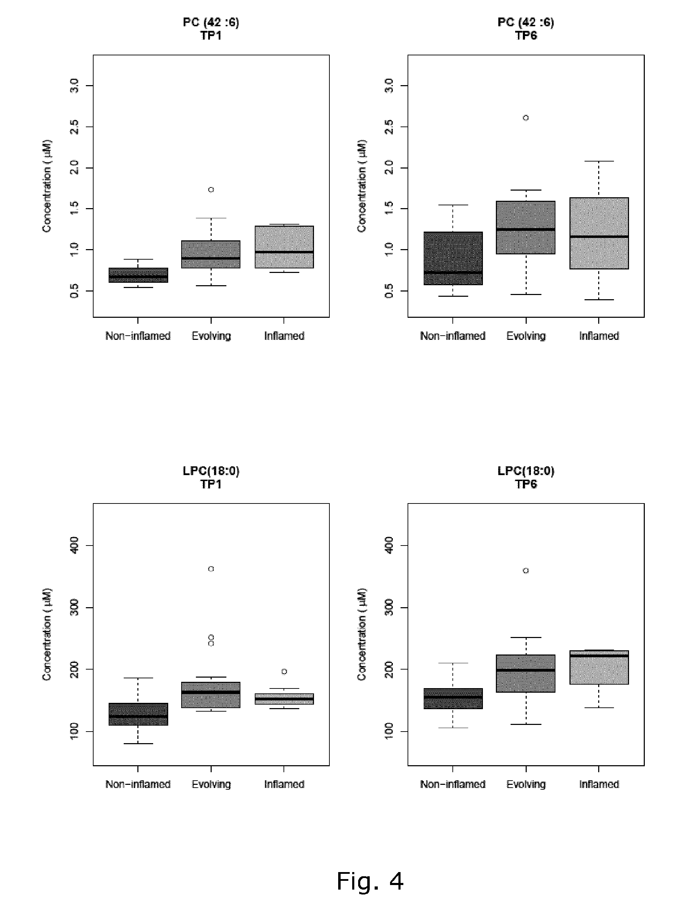

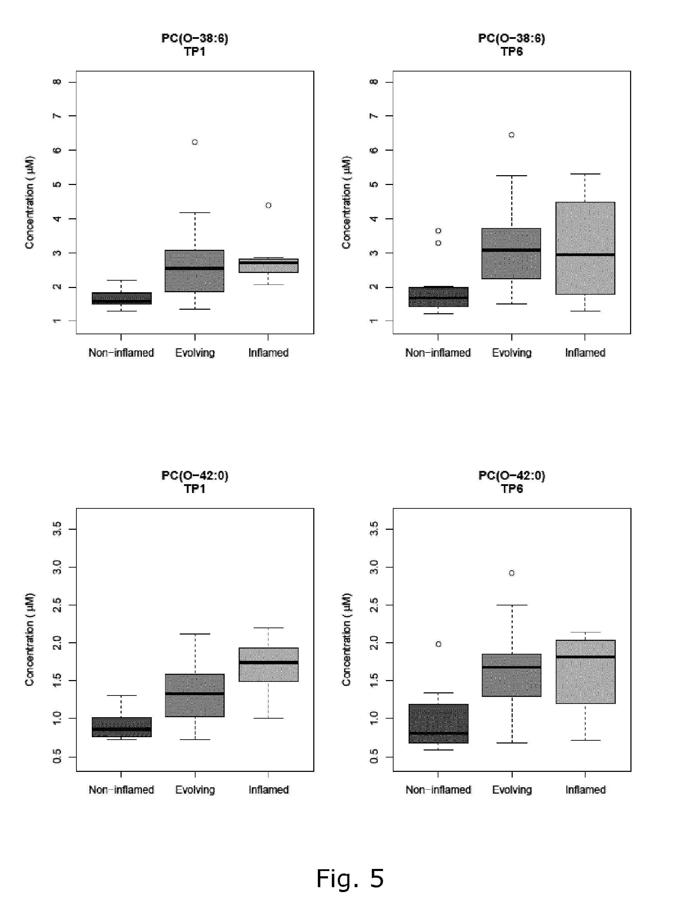

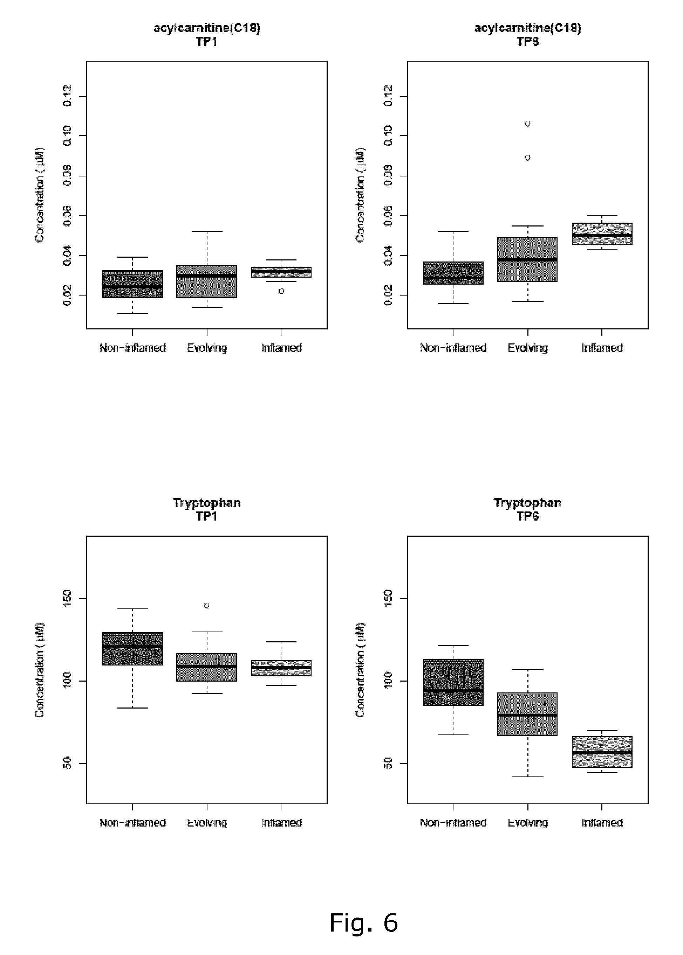

To verify the findings in the evolving animals group, changes in concentration of each of the 6 selected metabolites were evaluated for both permanently non-inflamed (NI) and inflamed (I) groups of animals. Simultaneously, these changes were also addressed between time points 1 and 6 (figures 4-6).

To further strengthen the interpretation, the P-values for each of the metabolites listed in table 1 at timepoint 1 and timepoint 6 were determined for non-inflamed versus inflamed and for the non-inflamed group versus the inflamed groups (table 2).

**Table 2: Summary of P-values calculated for time point (TP) 1 (non-inflamed (NI) vs. inflamed (I)) and for time point 6; and for non-inflamed (NI) (time point 1 versus 6) and for inflamed (I) groups.**

| | TP 1 non-inflamed *vs.* inflamed | TP 6 non-inflamed *vs*. inflamed | Non-inflamed TP1 *vs*. TP6 | Inflamed TP1 *vs*. TP6 |
|---|---|---|---|---|
| Metabolite | P-value | P-value | P-value | P-value |
| Tryptophan | 0.114430 | 0.002930 | 0.011582 | 0.006061 |
| PC(O-42:0) | 0.000476 | 0.137730 | 0.877690 | 0.927270 |
| PC (42:6) | 0.004962 | 0.571430 | 0.781810 | 0.890910 |
| LPC(18:0) | 0.026514 | 0.077656 | 0.064706 | 0.127270 |
| Acylcarnitine C18 | 0.134830 | 0.011722 | 0.217790 | 0.006061 |
| PC(O-38:6) | 0.000079 | 0.210990 | 0.600790 | 0.927270 |

### Conclusion

The data presented in figures 1-3 shows that it is possible to monitor the level of these biomarkers over time in order to identify specific trends of dysregulation (i.e. increase or decrease) leading towards low-grade inflammation.

The data presented in figures 4-6, from permanently non-inflamed and inflamed animals, shows that several metabolites have a stable concentration level at any time point for non-inflamed group while increasing concentration levels for inflamed one. Four metabolites revealed this behaviour: PC(O-42:0), PC(42:6), PC(O-38:6), acylcarnitine C18. Indeed, three phospholipids (PC(O-42:0), PC(42:6), PC(O-38:6) and one acylcarnitines (C18) showed an increased concentration at time point 6 for the inflamed rats while the median of the concentration values at time point 1 remained similar for the non-inflamed ones.

Aging may be a confounding factor associated to low-grade inflammation. In this context, biomarkers should reveal a different median concentration level at both time points for non-inflamed and inflamed groups. Two metabolites showed this behaviour: LPC(18:0) and Trp. Indeed, the non-inflamed group revealed an increased concentration of LPC(18:0) at both time points whereas the opposite for Trp. Such trend was confirmed for the inflamed group.

The data presented in table 2 shows the significance of the comparisons between rat groups (non-inflamed, inflamed) at each time point. The differences between time points for each group are also shown. Both aim to isolate the inflammatory behaviour from aging.

In conclusion, these results clearly show that the metabolites listed in table 1 are indeed early biomarkers for age-related low-grade inflammation.

## Claims

1. A method for predicting the risk of acquiring an age-related low-grade inflammation for a subject, said method comprising
a) providing a biological sample from a subject,
b) determining in said sample the level of at least one biomarker selected from the group consisting of
- a compound of molecular weight between 859-863 g/mol and which is an alkylacylphosphatidylcholine,
- a compound of molecular weight between 861-865 g/mol and which is a diacylphosphatidylcholine,
- a compound of molecular weight between 791-794 g/mol and which is an alkylacylphosphatidylcholine,
- a compound of molecular weight between 522-525 g/mol and which is a monoacylphosphatidylcholine,
- octadecanoylcarnitine (C18), and
- tryptophan,
c) comparing the level of the at least one biomarker to a reference level, and
d) determining whether said subject is likely to be at risk of acquiring an age-related low-grade inflammation, when the level of biomarker(s) deviate significantly from the respective reference level.

2. The method according to claim 1, wherein the one or more biomarkers are selected from the group consisting of a compound of molecular weight between 859-863 g/mol and which is an alkylacylphosphatidylcholine, a compound of molecular weight between 861-865 g/mol and which is a diacylphosphatidylcholine, a compound of molecular weight between 791-794 g/mol and which is an alkylacylphosphatidylcholine, a compound of molecular weight between 522-525 g/mol and which is a monoacylphosphatidylcholine, and octadecanoylcarnitine (C18),
whereby said subject is likely to be at risk of acquiring an age-related low-grade inflammation, if said determined one or more levels are significantly higher than the reference level and/or said subject is unlikely to be at risk of acquiring an age-related low-grade inflammation, if said determined one or more levels are equal to or lower than the reference level.

3. The method according to claim 1, wherein the biomarker is tryptophan,
whereby said subject is likely to be at risk of acquiring an age-related low-grade inflammation, if said determined the level is significantly lower than the reference level and/or said subject is unlikely to be at risk of acquiring an age-related low-grade inflammation, if said determined the level is equal to or higher than the reference level.

4. The method according to any of the preceding claims, wherein the level of at least two biomarkers are determined, such as level of at least three, such as level of at least four, such as level of at least five, or such as the level of all six biomarkers are determined.

5. The method according to any of the preceding claims wherein
- the compound of molecular weight between 859-863 g/mol and which is an alkylacylphosphatidylcholine is phosphatidylcholine (PC(O-42:0)), and/or
- the compound of molecular weight between 861-865 g/mol and which is a diacylphosphatidylcholine is phosphatidylcholine (PC(42:6)), and/or
- the compound of molecular weight between 791-794 g/mol and which is an alkylacylphosphatidylcholine is phosphatidylcholine (PC(O-38:6)), and/or
- the compound of molecular weight between 522-525 g/mol and which is a monoacylphosphatidylcholine is lysophosphatidylcholine (LPC(18:0)).

6. The method according to any of the preceding claims, wherein said biological sample is a body fluid such as blood plasma, whole blood, blood serum, saliva and urine, or a tissue sample such as a tissue biopsy.

7. The method according to any of the preceding claims, wherein the level of biomarker is the concentration of the biomarker in the biological sample.

8. The method according to any of the preceding claims, wherein the risk of acquiring an age-related low-grade inflammation is the risk within a period of 24 months from sampling, such as within 12 months, such as within 8 months, such as within 6 months, such as within 4 months, such as within 2 months, such as within 1 month.

9. The method according to any of the preceding claims, wherein the subject is a mammal such a human; a non-human species, including a primate; a livestock animal such as a sheep, a cows, a pig, a horse, a donkey, or a goat; a laboratory test animals such as mice, rats, rabbits, guinea pigs, or hamsters; or a companion animal such as a dog or a cat.

10. The method according to any of the preceding claims, wherein the determined risk is also the risk of acquiring one or more conditions defined by a low-grade inflammation, such as chronic low-grade inflammation.

11. The method according to claim 10, wherein the condition is selected from the group consisting of type 2 diabetes, hypertension, ischemic heart disease, atherosclerosis, Irritable Bowel Syndrome, Inflammatory Bowel Disease, psoriasis, cystic fibrosis, osteoporosis, osteoarthritis rheumatoid arthritis, sarcopenia, steatohepatitis, non alcoholic fatty liver disease, Alzheimer's disease, and Parkinson's disease.

12. The method according to any of the preceding claims, wherein the subject is a human more than 20 years of age, such as more than 30 years of age, such as more than 40 years of, such as more than 50 years of age, such as more than 60 years of age, or such as more than 70 years of age.

13. A method for determining the effect of a treatment for lowering the risk of acquiring an age-related low-grade inflammation for a subject, said method comprising
- providing a first biological sample from the subject, obtained before a treatment, and determining a first risk of acquiring an age-related low-grade inflammation according to any of claims 1-12,
- providing a second biological sample from the subject, obtained subsequent to the treatment or during the treatment, and determining a second risk of acquiring an age-related low-grade inflammation according to any of claims 1-12, and
- comparing the first risk to the second risk, thereby providing an estimate of the effect of the treatment.

14. The method according to claim 13, wherein
- a significantly increased risk in the second determined risk compared to the first determined risk is indicative of that the treatment is increasing the risk of acquiring an age-related low-grade inflammation, or
- an unchanged risk in the second determined risk compared to the first determined risk is indicative of that the treatment does not influence the risk of acquiring an age-related low-grade inflammation, or
- a significantly lower risk in the second determined risk compared to the first determined risk is indicative of that the treatment is lowering the risk of acquiring an age-related low-grade inflammation.

15. The method according to claim 13 or 14, wherein the treatment is a dietary treatment or a pharmaceutical treatment.
